# EUROPEAN PATENT APPLICATION

(11) **EP 3 366 766 A1**
(43) Date of publication of application: **29.08.2018**
(21) Application number: 16857460.6
(22) Date of filing: 19.10.2016
(51) Int. Cl.: C12M 3/00, B01L 1/00, C12M 1/00, F24F 7/06

(54) **CELL TREATMENT DEVICE**

(30) Priority: 20.10.2015 JP 2015206192
(71) Applicant: Rohto Pharmaceutical Co., Ltd., Osaka-shi Osaka 544-8666 (JP)
(72) Inventor: KOIKE, Tetsuo, Osaka-shi Osaka 544-8666 (JP); TAKIMOTO, Masahiro, Osaka-shi Osaka 544-8666 (JP); YAGI, Yoshiki, Kuse-gun Kyoto 613-0036 (JP)
(74) Representative: DTS Zürich
(86) International application number: PCT/JP2016/080942
(87) International publication number: WO 2017/069147

(57) **Abstract**

Provided is a cell treatment apparatus including an isolator 3 that is configured to treat cells in the inner space maintained in aseptic conditions, and a pass box 4 that is configured to carry an article for use in treatment of cells in the inside of the isolator 3 or a reagent container with a reagent therein into the inside of the isolator 3. The pass box 4 includes a clean bench chamber R1 that includes an opening and closing door to enable the article to be carried thereinto and decontaminate the article, and a decontamination chamber R2 that has a decontamination capability and is configured to be able to carry the article from the clean bench chamber R1 into the inner space of the decontamination chamber R2 and carry the article from the inner space of the decontamination chamber R2 into the inside of the isolator 3.

## Description

### CROSS-REFERENCE TO RELATED APPLICATION

This application claims priority to Japanese Patent Application No. 2015-206192, the disclosure of which is incorporated herein by reference in its entirety.

### FIELD

The present invention relates to a cell treatment apparatus that includes an isolator for cell treatment.

### BACKGROUND

In recent years, cell culture is performed using tissues and cells of various sites of human body, fertilized eggs, or the like, and the cultured cells have been put to practical use for regenerative medicine. In the cell culture, it is important to prevent contamination of cells by bacteria or the like during the culture. Therefore, a cell treatment apparatus that enables the culture of cells within a housing having a configuration that can maintain thereinside in aseptic conditions has been already proposed.

The aforementioned cell treatment apparatus includes, within a housing, an isolator that maintains thereinside in aseptic conditions and includes a mounting device such as gloves or a suit that is operated by an operator from the outside, a pass box, through which a culture vessel or the like is carried into the isolator, a clean box that forms an inner space for covering an insertion opening of the mounting device of the isolator or an opening and closing door, through which an article is carried into the pass box, and an air-lock chamber located on an inlet-side of the clean box that allows an article for cell treatment to be carried into the clean box in a state shut off from the outside (for example, Patent Literature 1).

### CITATION LIST

### Patent Literature

Patent Literature 1: JP 2014-198079 A (see Fig. 2)

### SUMMARY

### Technical Problem

In the aforementioned configuration of Patent Literature 1, it is necessary to install in the housing the clean box having such a large space as to enable the operator to enter the apparatus and operate a variety of containers within the isolator using the mounting device. In addition, it is necessary to have a configuration enabling all the chambers of the housing to be decontaminated in order to enable the operator to enter the clean box. Consequently, there is a disadvantage of increasing the entire size of the cell treatment apparatus.

In view of the above circumstances, it is an object of the present invention to provide a cell treatment apparatus that can suppress enlargement of the size of the apparatus itself.

### Solution to Problem

A cell treatment apparatus according to the present invention includes: an isolator that has an inner space maintained in aseptic conditions and is configured to treat cells in the inner space; a pass box that is configured to carry an article for use in treatment of cells in the inside of the isolator into the inside of the isolator; the pass box including a clean bench chamber and a decontamination chamber, the clean bench chamber having an opening and closing door and being configured to decontaminate the article carried thereinto through an opening formed by opening the opening and closing door, the decontamination chamber having a decontamination capability and an inner space and being configured to be able to carry the article from the clean bench chamber into the inner space of the decontamination chamber from the clean bench chamber and carry the article into the inside of the isolator.

The cell treatment apparatus according to the present invention may be configured so that the isolator includes an air supply unit that supplies air thereinto, the decontamination chamber includes an air supply unit that supplies air thereinto, the clean bench chamber includes a decontamination chamber opening that is openable and closable to carry the article into the decontamination chamber therethrough, the decontamination chamber includes an isolator opening that is openable and closable to carry the article into the isolator therethrough, and air flows from the decontamination chamber into the clean bench chamber through the decontamination chamber opening during it is opened, or air flows from the isolator into the decontamination chamber through the isolator opening during it is opened.

The cell treatment apparatus according to the present invention may be configured so that the clean bench chamber includes a decontamination chamber opening that is openable and closable to carry the article into the decontamination chamber therethrough, the decontamination chamber includes an air supply unit that supplies air thereinto and an air exhaust unit that exhausts air therefrom to the outside, and an isolator opening that is openable and closable to carry the article into the isolator therethrough, the supply of air into the decontamination chamber is maintained during the isolator opening is closed and the decontamination chamber opening is opened, and air supplied into the decontamination chamber flows toward the clean bench chamber through the decontamination chamber opening by stopping the exhaust of air from the decontamination chamber to the outside or decreasing the amount of air exhausted from the decontamination chamber to the outside.

The cell treatment apparatus according to the present invention may be configured so that the clean bench chamber includes a decontamination chamber opening that is openable and closable to carry the article into the decontamination chamber therethrough, the decontamination chamber includes an air supply unit that supplies air thereinto and an air exhaust unit that exhausts air therefrom to the outside, and an isolator opening that is openable and closable to carry the article into the isolator therethrough, the isolator includes an air supply unit that supplies air thereinto and an air exhaust unit that exhausts air therefrom to the outside, the exhaust of air from the isolator to the outside and the supply of air into the decontamination chamber are stopped or the amount of air exhausted from the isolator to the outside and the amount of air supplied into the decontamination chamber are decreased during the decontamination chamber opening is closed and the isolator opening is opened, and air supplied into the isolator flows toward the decontamination chamber through the isolator opening by maintaining the supply of air into the isolator and the exhaust of air from the decontamination chamber to the outside.

The cell treatment apparatus according to the present invention may be configured so that each of the isolator opening and the decontamination chamber opening has such a height as to allow an article having a largest dimension among the articles to pass therethrough.

### BRIEF DESCRIPTION OF DRAWINGS

Fig. 1 is a front view of an apparatus to produce cultured cell products of the present invention.
Fig. 2 is a plan view of the aforementioned production apparatus.
Fig. 3 is a view of the aforementioned production apparatus as seen from the outlet side.
Fig. 4 is an explanatory diagram showing the state immediately before a vessel cap is opened by a robot arm.
Fig. 5 is a view as seen from the direction of arrow A-A in Fig. 2.
Fig. 6 is a cross sectional view taken along line B-B in Fig. 2.
Fig. 7 is a cross sectional view taken along line C-C in Fig. 2.
Fig. 8 is a plan view showing a door slide structure included in a decontamination chamber of a pass box.
Fig. 9 is a view as seen from the direction of arrow D in Fig. 8.
Fig. 10 is a view as seen from the direction of arrow E in Fig. 8.
Fig. 11a is an explanatory diagram showing the air conditioning state before a packaging bag with a container therein is put into a clean bench chamber.
Fig. 11b is an explanatory diagram showing the state where the packaging bag with the container therein has been put in the clean bench chamber.
Fig. 12a is an explanatory diagram showing the state where the packaging bag with the container therein kept in the clean bench chamber has been put in a decontamination chamber.
Fig. 12b is an explanatory diagram showing the state where two containers have been taken out from the packaging bag put in the decontamination chamber.
Fig. 13a is an explanatory diagram showing the state where the container in the decontamination chamber has been put into the isolator.
Fig. 13b is an explanatory diagram showing the state where an opening for the isolator has been changed into the closed state from the state shown in Fig. 13a.

### DESCRIPTION OF EMBODIMENTS

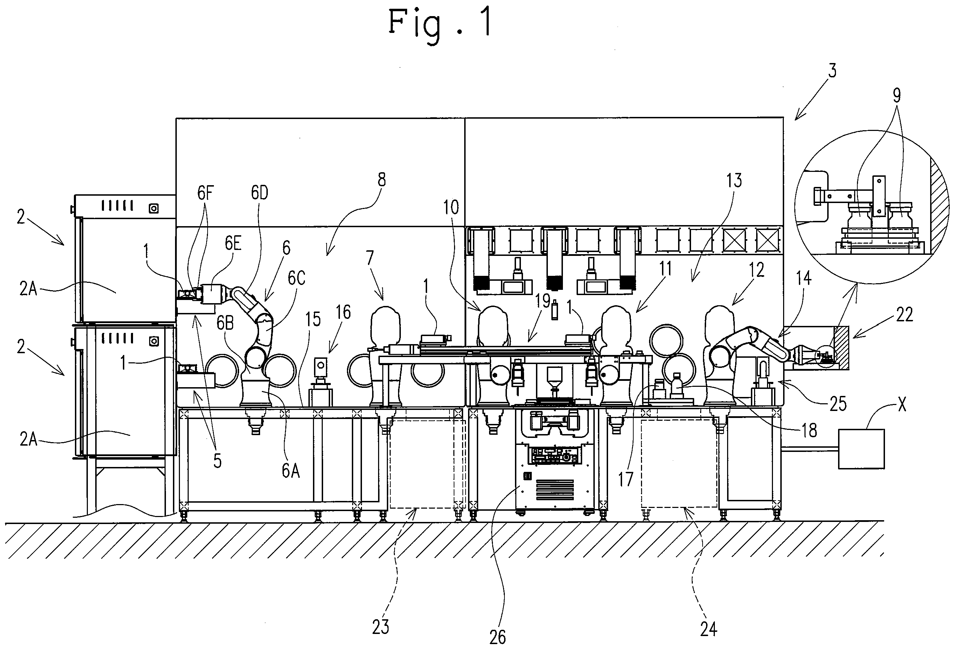

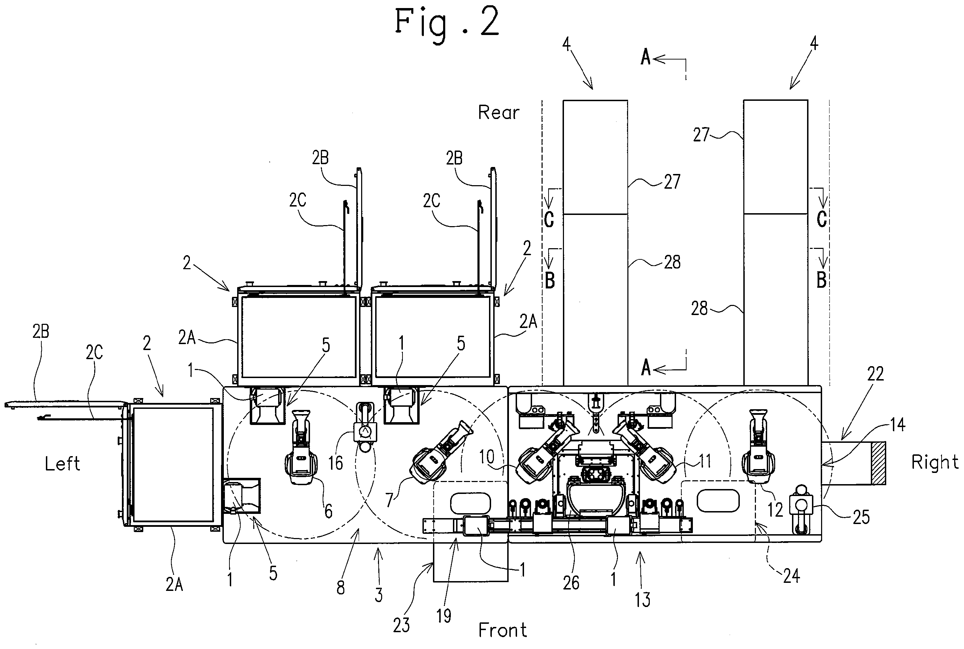

Hereinafter, an apparatus to produce cultured cell products (hereinafter, referred to as production apparatus) that is an example of a cell treatment apparatus of the present invention will be described. In the following description on the front, rear, left, and right directions, the left and right directions correspond to the state shown in Fig. 1 and Fig. 2, and the front and rear directions correspond to the state of Fig. 2 where the lower side corresponds to the "front" and the upper side corresponds to the "rear" (the directions are shown also in Fig. 2).

Fig. 1 to Fig. 3 show the production apparatus of this embodiment. The production apparatus includes a plurality of incubators 2, a horizontally elongated isolator 3, and a plurality (two in Fig. 2) of pass boxes 4. An incubator 2 houses a cell culture vessel (hereinafter referred to a culture vessel) 1 that is an article. The isolator 3 is capable of having its inside maintained in aseptic conditions and treats the culture vessel 1 transferred from the incubator 2. The pass boxes 4 are configured to be capable of carrying a reagent container into the isolator 3, in which the reagent container contains an article and a reagent necessary for subdividing and putting-in of cells cultured in the culture vessel 1. As the culture vessel 1, a HYPERFlask (manufactured by Corning Incorporated) capable of culturing cells in multilayers, for example, can be used. Each part is controlled by a control device X schematically shown in Fig. 1. The control device X may be provided integrally with the production apparatus or may be a separate body (such as a personal computer) connected to the production apparatus in a wired or wireless manner. Further, it is also possible to provide the control device X integrally with the production apparatus and provide only an operation section of the control device X that is operated by an operator (such as a tablet terminal) as a separate body from the production apparatus.

Six units of the incubators 2 are provided in total in a state of being vertically stacked in two stages as shown in Fig. 1, at three points in total including one point at the left end of the isolator 3 and two points in the left end part behind the isolator 3, as shown in Fig. 2. Two incubators 2 in the upper stage and the lower stage have the same configuration, and each of the incubators 2 is provided with racks (not shown) capable of housing a large number of the culture vessels 1 within a casing 2A. The casing 2A has a box shape with one lateral side opening so that the culture vessels 1 can be taken in and out through the lateral side. Two doors 2B and 2C configured to close the opening on the one lateral side of the casing 2A are attached to the casing 2A so as to be freely openable. The inside door 2C is formed with a transparent material, so that the number of the culture vessels 1 housed therein and the state of the cell culture can be checked only by opening the outside door 2B while the opening is closed by the inside door 2C. Further, carbon dioxide gas for adjusting the culture atmosphere is configured to be supplied into the incubators 2. Further, the culture vessels 1 housed on the racks inside the incubators 2 are configured to be delivered onto a plurality of mounting tables 5 provided in the isolator 3 by a delivery mechanism, which is not shown. Six units of the mounting tables 5, which is the same number as the number of the incubators 2, are arranged corresponding to the incubators 2.

As described above, the isolator 3 is horizontally elongated (in this embodiment, it is rectangular in planer view), where one set (two units on the upper and lower sides) of incubators 2 is located on the short side of the isolator 3 (in this embodiment, the left side), and a plurality of sets (in this embodiment, two sets of incubators 2) are located on the longitudinal side (in this embodiment, the rear side). This configuration can reduce the size of the production apparatus without decreasing the number of cultured cells.

The isolator 3 includes an observation section 8, a processing section 13, and an outlet 14. The observation section 8 includes two first robot arms 6 and 7 configured to move the culture vessels 1 to an observation position so that the degree of growth in the culture vessels 1 taken out of the incubators 2 is checked. The processing section 13 is provided continuously with the observation section 8. The processing section 13 includes three second robot arms 10, 11, and 12. The second robot arms 10, 11, and 12 are configured to transfer cells in the culture vessels 1 that have a specified number of cells out of the culture vessels 1 observed in the observation section 8 into a large number of product containers 9 (such as vial containers, see the enlarged view of Fig. 1), which are articles carried in from the pass boxes 4. The outlet 14 is configured to allow the large number of product containers 9 into which the cells have been transferred to be taken out therethrough. A large number of work gloves (not shown) that allow the operator to perform operations by putting their hands into the isolator 3 are attached onto the front and rear walls of the isolator 3. As shown in Fig. 2, the five robot arms 6, 7, 10, 11, and 12 are aligned in a straight line extending in the left and right directions along the longitudinal direction of the isolator 3.

With reference to the left and right directions, the first robot arm 6 on the left side corresponds to one set of incubators 2 located on the short side of the isolator 3 (in this embodiment, on the left side) and one set of incubators 2 on the left side out of the sets of incubators 2 located on the longitudinal side (in this embodiment, on the rear side). The first robot arm 6 on the left side can handle the culture vessels 1 that are housed in these incubators 2 (the range that can be reached by each robot arm (in planer view) is shown in Fig. 2 with a dashed-double-dotted circle). Further, the first robot arm 7 on the right side corresponds to one set of incubators 2 on the right side out of the sets of incubators 2 located on the longitudinal side of the isolator 3. The first robot arm 7 on the right side can handle the culture vessels 1 housed in the incubators 2.

With reference to the left and right directions, the second robot arm 10 on the left side and the second robot arm 11 in the middle correspond to the pass box 4 on the left side out of the pass boxes 4 located on the longitudinal side of the isolator 3 (in this embodiment, on the rear side). The second robot arm 10 on the left side and the second robot arm 11 in the middle can handle reagent containers in which articles and reagents are contained, the reagent containers being to be housed (or having been housed) in the pass box 4.

The second robot arm 12 on the right side corresponds to the pass box 4 on the right side out of the pass boxes 4 located on the longitudinal side of the isolator 3 (in this embodiment, on the rear side) and a box 22 for carrying out the product containers 9. The second robot arm 12 on the right side can handle reagent containers in which articles and reagents are contained, the reagent containers being to be housed (or having been housed) in the pass box 4, and the product containers 9 to be housed in the box 22.

As seen from the overlapping of the dashed-double-dotted circles shown in Fig. 2, the five robot arms 6, 7, 10, 11, and 12 are arranged in a positional relationship so as to be capable of passing articles to each other.

In this way, the robot arms 6, 7, 10, 11, and 12 are located within the isolator 3, thereby enabling each of the robot arms 6, 7, 10, 11, and 12 to act on the incubators 2, the isolator 3, the pass boxes 4, and the box 22 according to the purpose. Thus, according to the production apparatus of this embodiment, it is possible to improve the working efficiency and contribute to mass production of cultured cell products.

The first robot arms 6 and 7 and the second robot arms 10, 11, and 12 in this embodiment have the same configuration. Therefore, the description for the first robot arm 6 located at the left end will be applied to the description for each of the first robot arm 7, and the second robot arms 10, 11, and 12. The first robot arm 6 is constituted by articulated robot arm. The first robot arm 6 includes a fixed part 6A fixed to a base member 15 of the isolator 3, a base part 6B that is pivotable about the vertical axis at the distal end part of the fixed part 6A, a first arm 6C that is swingable about the horizontal axis at the distal end part of the base part 6B, a second arm 6D that is swingable about the horizontal axis at the distal end part of the first arm 6C, a third arm 6E that is swingable about the horizontal axis at the distal end part of the second arm 6D, and a pair of grips 6F that are attached to the distal end of the third arm 6E so as to be opposed thereto. The pair of grips 6F are configured to be capable of moving close to and away from each other. The articulated first robot arms 6 and 7 can hold the culture vessels 1 delivered from the incubators 2 using the pair of grips 6F (see Fig. 1) and move them to a microscope 16 at the observation position. The second robot arms 10, 11, and 12 are configured to hold such as a centrifuge tube 17 and a preparation tank 18 shown in Fig. 1 in addition to the culture vessels 1 so as to be capable of performing various processes.

The microscope 16 is arranged at the observation position between the two first robot arms 6 and 7. By arranging the microscope 16 as above, it is possible to move the culture vessels 1 to the microscope 16 using the first robot arm 6 on the left side so as to observe the cells, and as a result of the observation, it is possible to hold the culture vessels 1 that have been determined to have a specified number of cells so as to rapidly move them to the processing section 13 side, using the first robot arm 7 on the right side. The first robot arm 6 on the left side mainly performs the operation to move the culture vessels 1 to the microscope 16, and the first robot arm 7 on the right side performs the operation to move the culture vessels 1 that have been determined to have a specified number of cells toward the processing section 13 side. These operations by the first robot arms 6 and 7 can accelerate the operation speed. The determination on whether the culture vessels 1 have a specified number of cells may be made by counting the number of cells by visual inspection of the operator (human) of the production apparatus or may be made by the control device based on the number of cells calculated by analyzing an image captured by a camera so as to calculate the number of cells. The culture vessels 1 that are delivered from the incubator 2 located opposed to the first robot arm 7 on the right side are held by the first robot arm 7 on the right side to be moved to the microscope 16. Further, a microscope 25 is provided also in the processing section 13. The object observed by the microscope 25 is held by the second robot arm 12 on the right end to be moved.

The culture vessels 1 after the observation are conveyed not only by being directly passed from the first robot arm 7 on the right side to the second robot arms 10 arranged at the left end of the processing section 13. For example, in the case where the second robot arm 10 is in an operation, the culture vessels 1 are conveyed by a conveying apparatus 19 to a position where the second robot arm 10 at the left end of the processing section 13 or the second robot arm 11 arranged at horizontal center of the processing section 13 can grip them. The conveying apparatus 19 is provided along the front sidewall of the isolator 3 and is set to a length that allows the conveying apparatus 19 to convey them from the right end part of the observation section 8 of the isolator 3 to the horizontal center of the processing section 13. Accordingly, when the first robot arm 7 on the right side passes the culture vessels 1 after the observation to the conveyance starting end part of the conveying apparatus 19, the conveying apparatus 19 conveys the culture vessels 1 to the position where one of the two second robot arms 10 and 11 can grip them.

The conveying apparatus 19 is provided corresponding to at least one robot arm (in this embodiment, the first robot arm 7) located in the observation section 8 and a plurality of robot arms (in this embodiment, the two second robot arms 10 and 11) located in the processing section 13. The first robot arm 7 can directly deliver the articles to the second robot arm 10. The conveying apparatus 19 can deliver the articles to the first robot arm 7 and the third robot arm 11 between which direct delivery of the articles is impossible. Therefore, even in the case where the articles cannot be delivered from the first robot arm 7 to the third robot arm 11 via the second robot arm 10 due to the second robot arm 10 being in operation, the articles can be delivered from the first robot arm 7 to the third robot arm 11 via the conveying apparatus 19. Therefore, the articles can be conveyed in parallel (via a plurality of routes) within the isolator 3. Accordingly, the working efficiency within the isolator 3 can be improved, and thus the productivity can be improved.

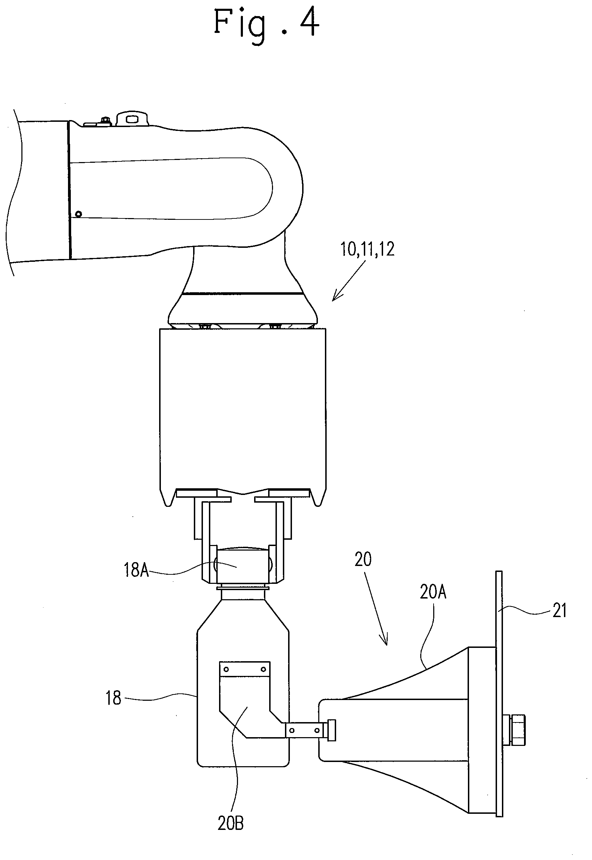

In the processing section 13, three units of the second robot arms 10, 11, and 12 are arranged at equal intervals, and the intervals are set to be smaller than the interval between the two first robot arms 6 and 7, so that the speed of various processes performed between the second robot arms 10 and 11 or 11 and 12 is higher. As shown in Fig. 4, an immovable fixed auxiliary arm 20 is provided at a position in the vicinity of each of the second robot arms 10, 11, and 12 and below each of the second robot arms 10, 11, and 12. The auxiliary arm 20 includes a fixed part 20A fixed to a fixing member 21, and a pair of grips 20B (in Fig. 4, only the grip 20B on the front side is shown) attached so as to be capable of moving close to and away from the fixed part 20A. In Fig. 4, for example, after the upper end part of the preparation tank 18 is held by the second robot arm 10, 11, or 12, so as to be moved to a position where it can be gripped by the pair of grips 20B of the auxiliary arm 20, the lower end part of the preparation tank 18 is gripped by the pair of grips 20B of the auxiliary arm 20. In this way, a cap 18A of the preparation tank 18 can be opened or closed by the single second robot arm 10, 11, or 12. Further, a program to open and close screw caps that are provided on a plurality of types of containers is stored in the second robot arms 10, 11, and 12, so that the second robot arms 10, 11, and 12 can open and close the screw caps provided on the plurality of types of containers. Therefore, it is not necessary to unify the types of containers into the same type, and thus the production apparatus of cultured cell products can be easily achieved. Also in the first robot arms 6 and 7, such a program may be stored.

The two pass boxes 4 are provided to be continuous with the rear wall of the processing section 13. One (on the left side) of the pass boxes 4 is arranged so that the articles can be carried therein passing through between the second robot arm 10 located at the left end and the second robot arm 11 located at the center. Examples of the articles include a plurality of types of containers including the product containers 9, the culture vessels 1, and the centrifuge tube 17, and the preparation tank 18 that is a container in which drugs are put. The other (on the right side) of the pass boxes 4 is arranged so that the articles are carried to the second robot arm 12 located at the right end.

As described above, the isolator 3 is horizontally elongated, in which the plurality (in this embodiment, two) of pass boxes 4 are located on the longitudinal side of the isolator 3 (in this embodiment, on the rear side). This configuration can reduce the size of the production apparatus without limiting the amount of articles to be carried into the isolator 3.

The opening of the outlet 14 is configured to have a size such that the second robot arm 12 located at the right end can easily enter therethrough. The outlet 14 is provided with a freely openable electric shutter (not shown) and is provided continuously with the box 22 that forms a space in which the product containers 9 moved through the outlet 14 to the outside of the isolator 3 are kept for a while.

The processing section 13 includes a first transfer processing unit, a separation processing unit, and a second transfer unit. The first transfer processing unit is configured to transfer a cell-containing liquid housed in the culture vessels 1 received from the first robot arm 7 into the centrifuge tube 17 using the second robot arm 10. The separation processing unit is configured to separate the cells and a liquid portion by subjecting the centrifuge tube 17 to a centrifuge 26 using the second robot arm 10. The second transfer unit is configured to transfer a specified number of cells within the centrifuge tube 17 into a large number of the product containers 9 while a preservative solution (cryopreservation solution) is put into the centrifuge tube 17 after removing at least part of the liquid portion separated in the separation processing unit from the centrifuge tube 17, using the second robot arm 10. In the description of this embodiment, the term "cell-containing liquid" simply means a "liquid containing cells" and is not limited to a liquid in a specific state.

The processing section 13 includes a medium-replacing unit configured to replace the culture medium within the culture vessels 1 taken out of the incubators 2 using the first robot arm 7. The medium-replacing unit is configured to open the caps of the culture vessels 1 received by the second robot arm 10 from the first robot arm 7, to dispose of the culture medium within the culture vessels 1, to supply another culture medium into the cell culture vessels 1, to put the caps thereon, and to return them to the first robot arm 7.

The processing section 13 configured as above is capable of performing a first process of thawing frozen cells and seeding them, a second process (passage process) of collecting the cells and seeding them on a large number of culture vessels, and a third process of collecting the cultured cells in the culture vessels after the passage process, subdividing the collected cells, transferring them into the product containers 9, and carrying them out through the outlet 14.

Provided at a position close to the processing section 13 within the observation section 8 is a large-size waste disposal part 23 for disposal of large-size waste products such as the centrifuge tube 17 and the preparation tank 18, in addition to the cell culture vessels 1, which become unnecessary during the aforementioned processes. Provided at a position close to the box 22 within the processing section 13 is a small-size waste disposal part 24 for disposal of small size waste products such as pipette tips (suction openings mounted to pipettes, not shown).

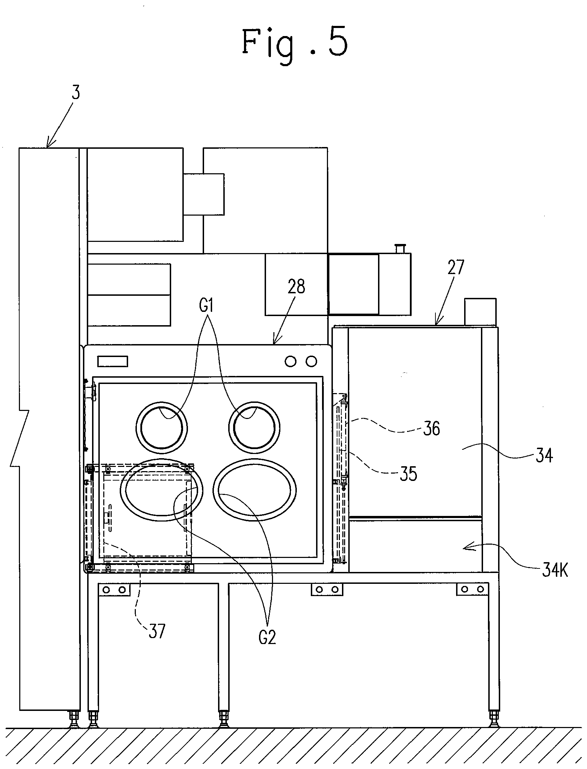

The two pass boxes 4 have the same configuration. Each of the pass boxes 4 includes a first box 27 that constitutes a clean bench chamber R1 for carrying containers from outside thereinto, and a second box 28 that constitutes a decontamination chamber R2 for carrying containers from the clean bench chamber R1 into the isolator 3. As shown in Fig. 5, each of inner side surfaces respectively opposed to the second boxes 28 (only one of them are shown in Fig. 5) has a large number of (four in Fig. 5) glove ports G1 and G2. Although not shown, a rack may be provided in an upper space of each of the first box 27 and the second box 28 so as to temporarily place thereon articles carried into the inside, which enables efficient container carrying-in operation from the clean bench chamber R1 to the decontamination chamber R2, and from the decontamination chamber R2 to the isolator 3. Although not shown, pipes for transferring a washing liquid or the like for use in the isolator 3 to the isolator 3, and pipes for discharging the washing liquid used in the isolator 3 are arranged in the pass box 4.

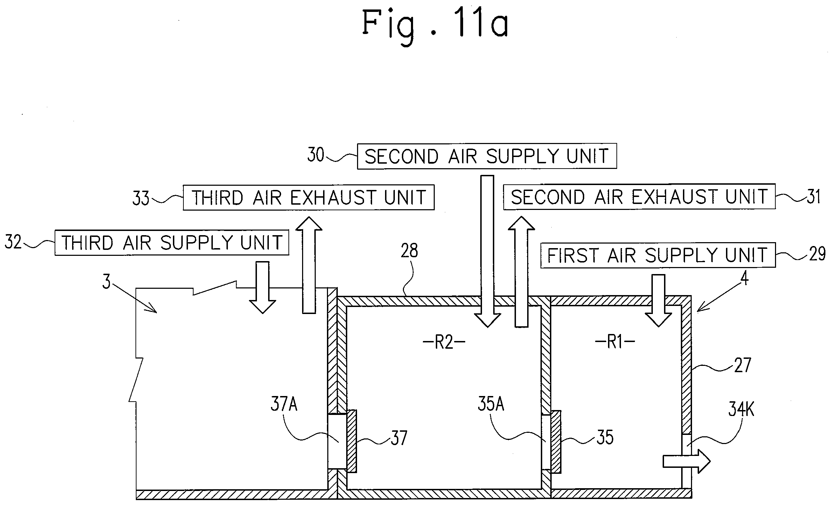

As shown in Fig. 11a, the clean bench chamber R1 includes a first air supply unit 29 that is constituted by an air supply fan for supplying air into the inside thereof. The decontamination chamber R2 includes a second air supply unit 30 that is constituted by an air supply fan for supplying air into the inside thereof, and a second air exhaust unit 31 that is constituted by an air exhaust fan for exhausting air to the outside. The isolator 3 includes a third air supply unit 32 that is constituted by an air supply fan for supplying air into the inside thereof, and a third air exhaust unit 33 that is constituted by an air exhaust fan for exhausting air to the outside. The isolator 3 and the decontamination chamber R2 is configured so that hydrogen peroxide gas is supplied thereinto from a hydrogen peroxide gas generating device (not shown). The description herein refers to hydrogen peroxide gas, but alternatively, ozone, chlorine dioxide gas, ethylene oxide gas or the like may be supplied.

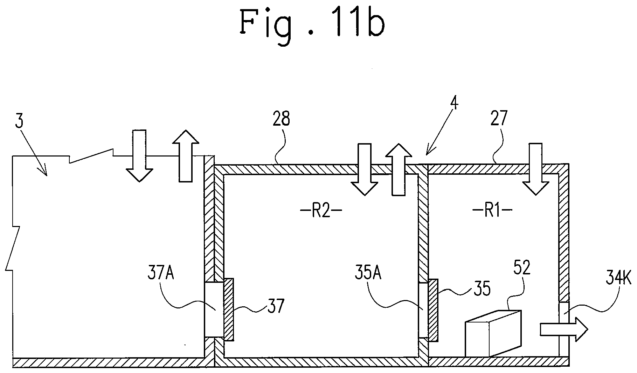

As shown in Fig. 5, shutters 34 (only one of them is shown in Fig. 5) as opening and closing doors of sliding type that can slide in the vertical direction are each provided on one lateral side of each of the two opposed clean bench chambers R1. Each of the shutters 34 is configured to change the opening degree of an opening 34K formed on a lower side. Herein, the opening 34K is set to be substantially one fourth of an area when the shutter 34 is in the fully opened position. Containers are manually passed through the opening 34K from the outside of the clean bench chamber R1 into the clean bench chamber R1, where containers which cannot be decontaminated are decontaminated with alcohol within the decontamination chamber R2. Decontamination treatment is performed in this way. Therefore, containers which can be decontaminated in the decontamination chamber R2 are not decontaminated in the clean bench chamber R1, but are carried into the decontamination chamber R2 where decontamination is performed. The size of a space for performing the operations in the clean bench chamber R1 is enough if it allows a largest size of containers to be subjected to decontamination treatment. In Figs. 11 to Figs. 13, the opening 34K is formed not on the one lateral side of the opposed clean bench chambers R1, but on one end side in the longitudinal direction of the pass box 4 (right end in Figures) in order to be able to illustrate flows of air.

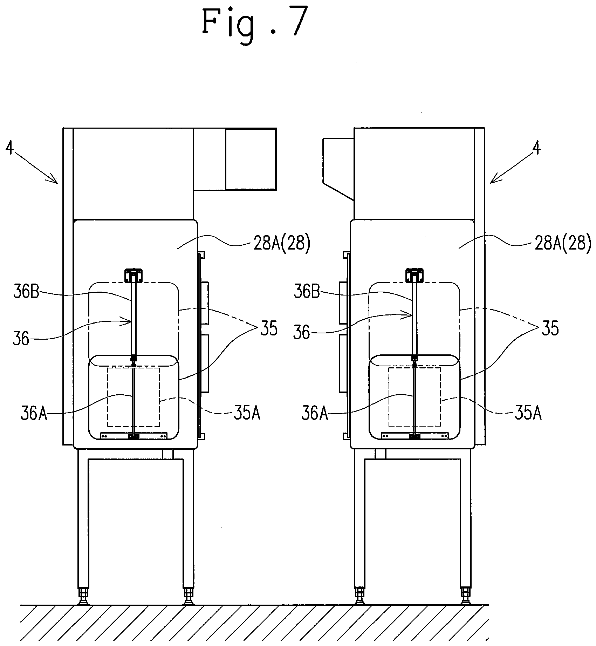

As shown in Fig. 7, Fig. 11a, and Fig. 11b, the clean bench chamber R1 includes a decontamination chamber opening 35A that is openable and closable for carrying containers into the decontamination chamber R2. This decontamination chamber opening 35A can be opened and closed by moving up and down a first cover 35 using an air cylinder 36. The first cover 35 is formed by a plate-shaped member having a substantially square shape, and is vertically movably supported to a side wall 28A of the second box 28 by a guide mechanism (not shown). The air cylinder 36 has a cylinder tube 36B that is fixed to the side wall 28A of the second box 28 with a proximal end of the cylinder tube 36B upwardly oriented, and has a piston rod 36A that is connected to a lower end of the first cover 35 with a distal end of the piston rod 36A oriented downwardly. The piston rod 36A of the air cylinder 36 is retracted to slide the first cover 35 upwardly so that the decontamination chamber opening 35A is brought into an opened state (see a two-dot chain line in Fig. 7), and the piston rod 36A of the air cylinder 36 in the retracted state is extended to slide the first cover 35 downwardly, which has been slid upwardly, so that the decontamination chamber opening 35A is brought into a closed state (see a solid line in Fig. 7).

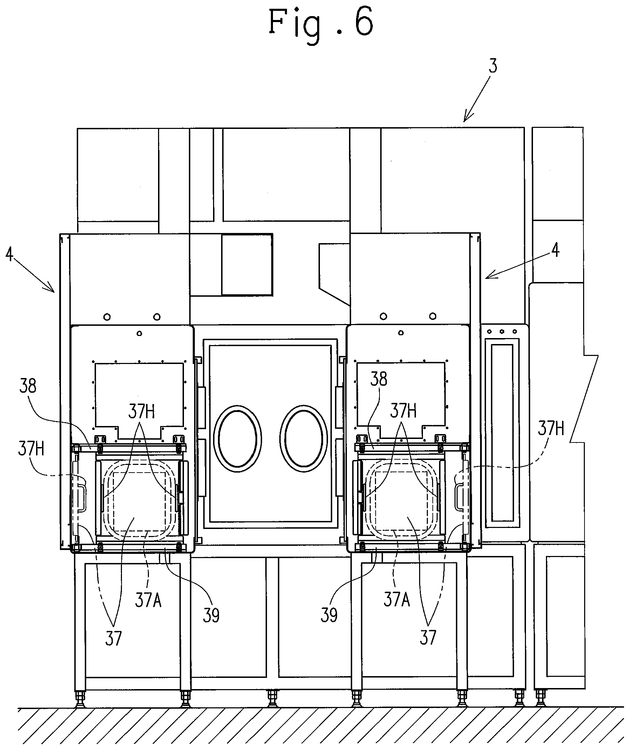

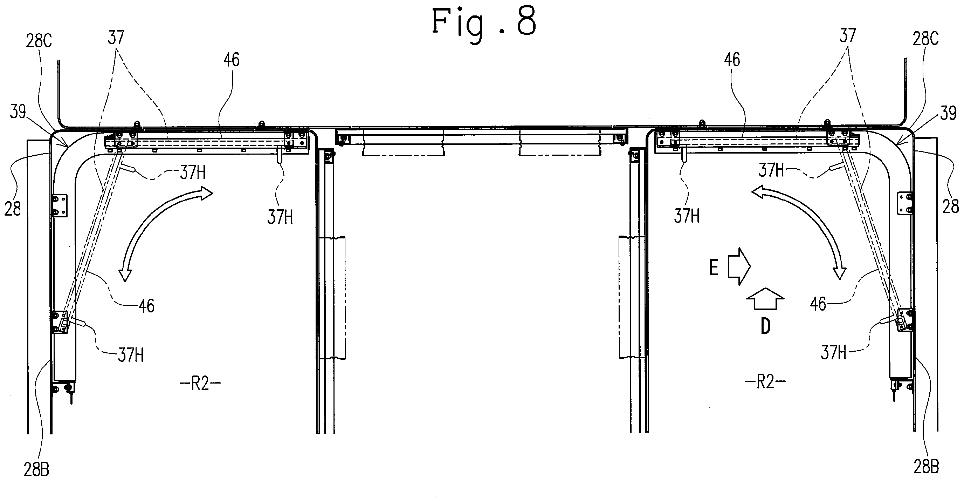

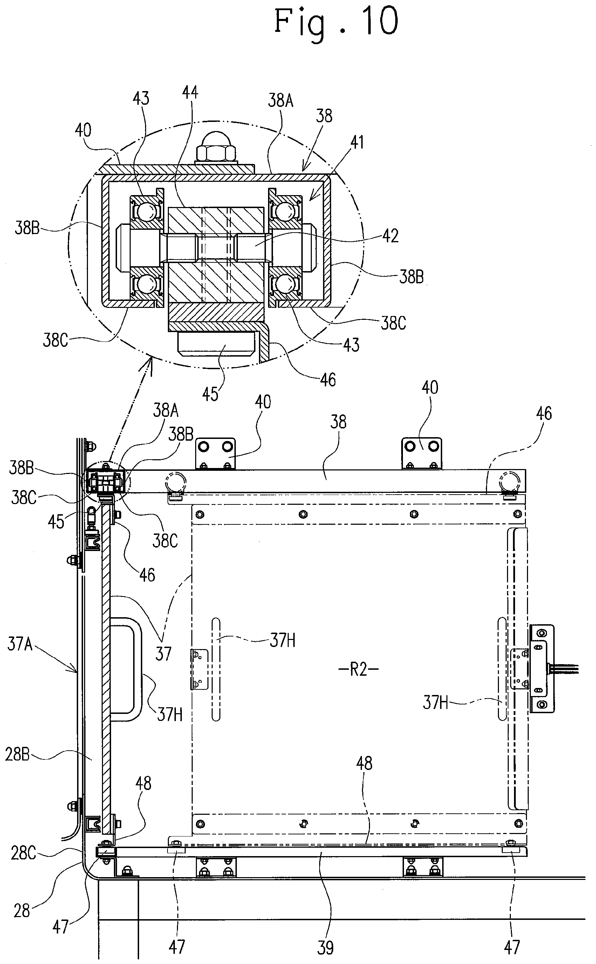

As shown in Fig. 6, Fig. 11a, and Fig. 11b, the decontamination chamber R2 includes an isolator opening 37A that can be opened and closed, through which containers carried from the clean bench chamber R1 into the decontamination chamber R2 is carried into the isolator 3. The isolator opening 37A is covered by a second cover 37, which slides in the lateral side to be able to open and close the isolator opening 37A. The second cover 37 is formed by a plate-shaped member having a substantially square shape, and includes a pair of left and right handles 37H for opening and closing operation at both ends in the slide direction. The second cover 37 is configured so as to be able to move in the lateral direction while having its upper and lower ends supported by a pair of upper and lower guide rails 38 and 39. In detail, as shown in Fig. 8 to Fig. 10, the upper guide rail 38 and the lower guide rail 39 are arranged to oppose to each other in the vertical direction, and are formed respectively by L-shaped members fixed to two adjacent side walls 28B and 28C of the second box 28 constituting the decontamination chamber R2. The upper guide rail 38 includes a top plate part 38A fixed to four brackets (only three brackets are shown in Figs. 11) fixed to the two side walls 28B and 28C, a pair of vertical plate parts 38B extending downward from both ends in the width direction of the top plate part 38A, and a pair of horizontal plate parts 38C that respectively extend from lower ends of the pair of vertical plate parts 38B to close to each other. A pair of moving bodies 41 are movably provided inside the upper guide rail 38, and an upper end of the second cover 37 is connected to the pair of moving bodies 41. Each of the moving bodies 41 includes a pair of bearings 43 that are mounted on the pair of horizontal plate parts 38C and coupled to each other through lateral shafts 42, a spacing member 44 for holding a space between the bearings 43 and having the lateral shafts 42 extending through the spacing member 44, and a pin 45 that extends through the spacing member 44 movably around a vertical axis and is attached to the spacing member 44. In each of the moving bodies 41, an upper frame body 46 having a reversed L-shape, which is fixed to an upper end of the second cover 37 with screws, is fastened and fixed to the spacing member 44 by the pin 45. Accordingly, the second cover 37 can smoothly change its moving direction without deflecting during moving through a curved portion of the upper guide rail 38 since the second cover 37 can smoothly move along the upper guide rail 38 by the rotation of the pair of bearings 43, while rotating around the axis of the pin 45. The lower guide rail 39 has a substantially U-shape with its upper end opening, and a pair of left and right rollers 47, which are rotatable around the vertical axis, are provided within the lower guide rail 39. The pair of left and right rollers 47 are coupled, rotatably around the vertical axis, to a lower frame body 48 that has a L-shape and is fixed to a lower end of the second cover 37 with screws. Accordingly, the second cover 37 can smoothly change its moving direction without deflecting during moving through a curved portion of the lower guide rail 39 since the second cover 37 can smoothly move along the lower guide rail 39 by the rotation of the pair of left and right rollers 47. In Fig. 10, the second cover 37, which is located at a position at which it closes the isolator opening 37A, is shown in solid line, and the second cover 37, which is located at a position at which it opens the isolator opening 37A, is shown in dashed-double-dotted line.

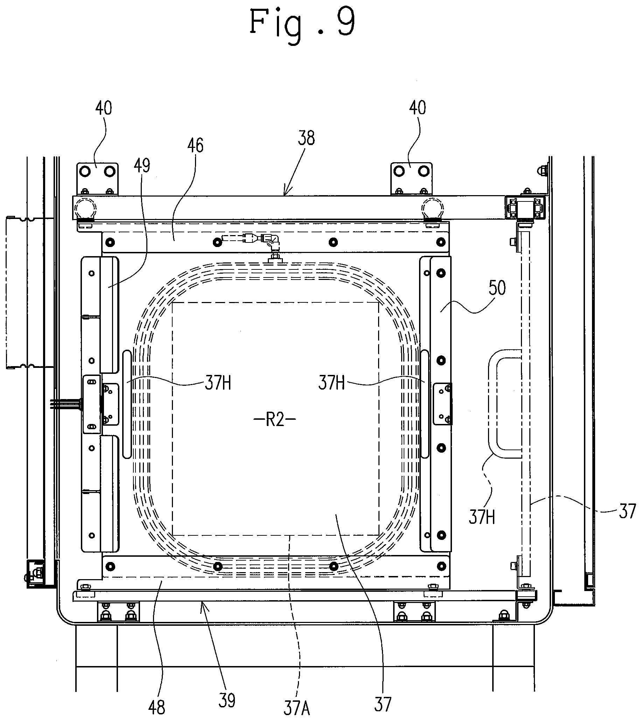

The reference numeral 49 shown in Fig. 9 represents a stopper member, to which the second cover 37 abuts when it is located at the closing position, and the reference numeral 50 represents a stopper member, to which the second cover 37 abuts when it is located at the opening position.

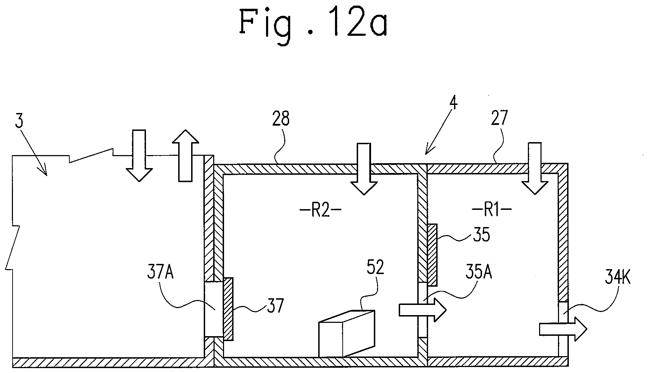

The description will be made for the procedures, in which containers 51 having the above configuration are made to pass through the pass box 4 and carried into the isolator 3. Here, the containers 51 are placed in a packaging bag 52. The packaging bag 52 with the containers 51 placed therein are put in the pass box 4, and thereafter, the containers 51 are taken out of the packaging bag 52, and the containers 51 taken out therefrom are carried into the isolator 3. Fig. 12a shows the packaging bag 52 just before carrying-in. Specifically, the first cover 35 and the second cover 37 are located at the closing positions, and supplying air by the third air supply unit 32 and exhausting air by the third air exhaust unit 33 are performed within the isolator 3. In the same manner as this, supplying air by the second air supply unit 30 and exhausting air by the second air exhaust unit 31 are performed within the decontamination chamber R2. Only the supply of air by the first air supply unit 29 is performed within the clean bench chamber R1. Clean air supplied into the clean bench chamber R1 is exhausted from the opening 34K (which is different in forming position from the opening 34K of Fig. 6, as described before) to the outside so as to prevent outside air from intruding into the clean bench chamber R1. In Figures other than Fig. 12a, the description for the air supply units 29, 30, and 32, and the air exhaust units 31 and 33 are omitted.

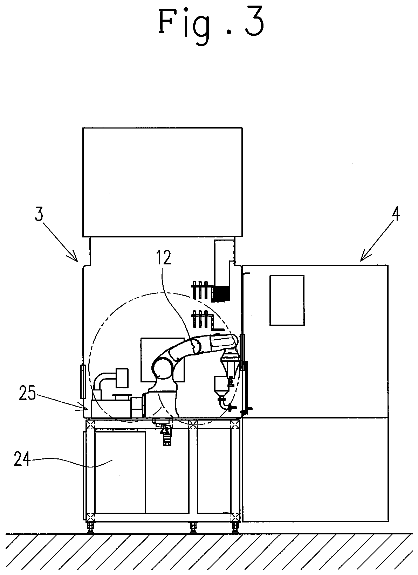

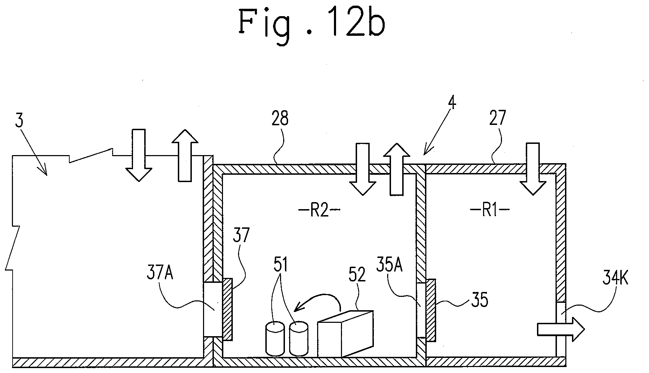

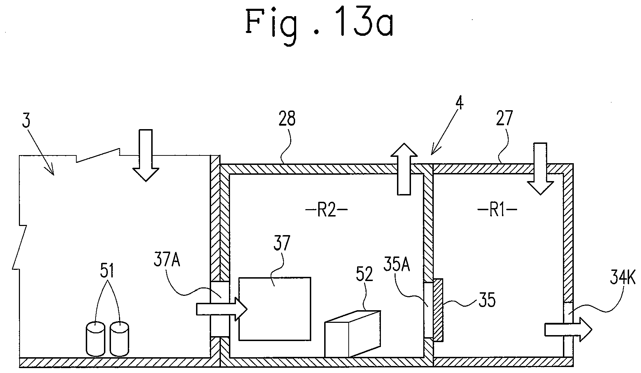

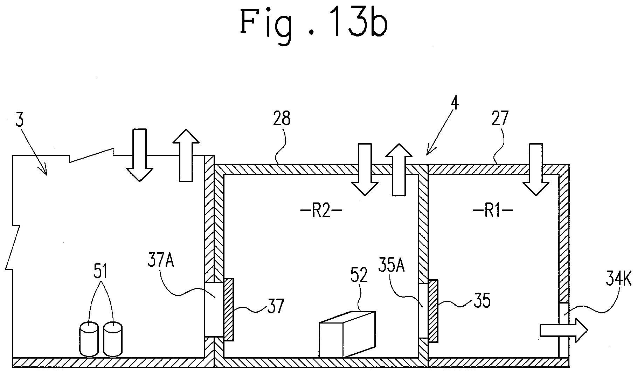

As shown in Fig. 12b, the state of which is different from the state shown in Fig. 12a, the operator carries the packaging bag 52 with the containers placed therein into the clean bench chamber R1 through the opening 34K. After the carrying in of the packaging bag 52, the operator passes the packaging bag 52 through the opening 34K, and wipes off the outer surface of the packaging bag 52 with alcohol containing cloth to subject the same to the alcohol decontamination. Next, the operator stops exhausting air from the decontamination chamber R2 to the outside, or decrease the amount of air exhausted from the decontamination chamber R2 to the outside from the amount of air exhausted in normal exhausting operation, and thereafter presses a switch (not shown) to locate the first cover 35 at the opening position. At this time, air supplied into the decontamination chamber R2 flows toward the clean bench chamber R1 through the decontamination chamber opening 35A and therefore air (contaminant) within the clean bench chamber R1 does not intrude into the decontamination chamber R2. The operator carries the packaging bag 52 within the clean bench chamber R1 into the decontamination chamber R2 through the decontamination chamber opening 35A (see Fig. 13a) under this circumstance. The operator, after carrying in, locates the first cover 35 at the closing position and thereafter restarts the exhaust of air, which has been stopped, or increases the amount of exhausted air, which has been decreased, to the normal amount of air exhausted before the reduction. Subsequently, the operator takes out two containers 51 from the packaging bag 52 (see Fig. 13b). The two containers 51 are packaged within the packaging bag 52 in aseptic conditions and therefore they are still held in aseptic conditions when they are taken out of the packaging bag 52. Subsequently, the operator stops the exhaust of air from the isolator 3 to the outside or decrease the amount of air exhausted from the isolator 3 to the outside to the amount smaller than the amount of air exhausted in normal exhaust operation, and at the same time stops the supply of air to the decontamination chamber R2 or decrease the amount of air supplied to the clean bench chamber R1 to the amount smaller than the amount of air supplied in normal supply operation. In this state, the operator grabs the handle 37H of the second cover 37 to slidingly move the second cover 37 in the lateral direction to the opening position. At this time, the supply and exhaust of air is controlled so that air to be supplied to the isolator 3 passes through the isolator opening 37A, flows toward the decontamination chamber R2, and is exhausted to the outside of the decontamination chamber R2. Thus, air (contaminant) within the decontamination chamber R2 does not intrude into the isolator 3. In this state, the operator carries the containers 51, which are being held in aseptic conditions, into the isolator 3 through the isolator opening 37A (see Fig. 3a). After the carrying-in, the operator holds the handle 37H of the second cover 37 to slidingly move the second cover 37 in the lateral direction to the closing position. Then, the operator restarts the exhaust of air from the isolator 3 and the supply of air into the decontamination chamber R2, or increase the amount of air exhausted from the isolator 3 to the outside and the amount of air supplied into the decontamination chamber R2, which have been decreased, to the amount of exhausted air and the amount of supplied air in normal operation before the reduction to finish the operation. When the amount of exhausted air and the amount of supplied air are to be increased or decreased (changed), it is conceivable to change the number of rotation of a motor of each of the air supply fan and the air exhaust fan, or adjust the opening degree of a damper for air flow adjustment.

Each of the isolator opening 37A and the decontamination chamber opening 35A has such a height as to allow a container having a largest dimension (herein HYPERFlask manufactured by Corning Incorporated) capable of culturing cells to pass therethrough. The opening 34K of the clean bench chamber R1 also has such a height as to allow a container having a largest dimension (herein HYPERFlask manufactured by Corning Incorporated) capable of culturing cells to pass therethrough. Such height setting enables the size of each opening to be minimized and air flows to be desirably controlled, while enabling all the kinds of handled containers (articles) to be carried into the respective chambers. As a result, it is possible to more securely avoid occurrence of troubles such as contamination.

The cell treatment apparatus according to the present invention is not limited to the aforementioned embodiment, and various modifications can be made without departing from the gist of the present invention.

The aforementioned embodiment was described by taking, for example, the case where the two robot arms 6 and 7 are provided in the observation section 8, and the three robot arms 10, 11, and 12 are provided in the processing section 13. However, it is also possible to apply, to the present invention, the configuration of providing at least one robot arm in the observation section 8 and providing at least one robot arm in the processing section 13.

In the aforementioned embodiment, the isolator 3 is configured to have a horizontally elongated shape as an example, but may be configured to have a square shape or a circular shape. Further, it may be configured to have a bent shape.

The aforementioned embodiment was described by taking, for example, the case where the first cover 35 is configured to be movable in the vertical direction and the second cover 37 is configured to be movable in the lateral direction. However, the moving direction of each of the first cover 35 and the second cover 37 may be set to any direction.

The description of the aforementioned embodiment was made for the apparatus to produce cultured cell products, which is configured to culture cells and subdivide cultured cells into products. However, the present invention is also applicable to an apparatus to culture cells, which is configured to perform only cell culturing, or applicable to a product manufacturing apparatus, which is configured to subdivide cultured cells into products.

The aforementioned embodiment was described by taking, for example, the case where the two pass boxes 4 are provided. However, the present invention is also applicable to the case where one pass box, or three or more pass boxes are provided.

The aforementioned embodiment was described by taking, for example, the case where each of the isolator 3 and the decontamination chamber R2 is provided with an air supply unit and an air exhaust unit. However, the present invention is also applicable to the case where only the air supply unit for supplying air is provided.

The configuration and action of the aforementioned embodiment will be summarized below. The cell treatment apparatus according to the embodiment includes: an isolator 3 configured to treat cells in the inside of the isolator 3 maintained in aseptic conditions; pass boxes 4 configured to carry an article for use in treatment of cells in the inside of the isolator 3 into the inside of the isolator 3, each of the pass boxes 4 including a shutter 34; a clean bench chamber R1 configured to decontaminate the article carried thereinto through an opening 34K formed by opening the shutter 34; and a decontamination chamber R2 that has a decontamination capability and is configured to be able to carry the article from the clean bench chamber R1 into the inside of the decontamination chamber R2 and carry the same into the inside of the isolator 3.

According to the above configuration, the openable and closable shutter 34 is opened to carry an article into the clean bench chamber R1 from the outside to operate decontamination treatment from the outside. Thus, the clean bench chamber R1 has such an inside space as to enable the operation for decontamination treatment from the outside, and the article before carried into the decontamination chamber R2 is subjected to decontamination treatment so that it is possible to decontaminate in aseptic conditions the article carried from the outside, while suppressing enlargement of the size of the apparatus itself. The temporal placement of the article, which has been decontaminated in the clean bench chamber R1, in the decontamination chamber R2 enables the article to be carried into the isolator 3 while maintaining the decontaminated conditions. Whereby, it is possible to suppress contamination inside of the isolator 3.

The cell treatment apparatus according to the embodiment may be configured so that the isolator 3 includes an air supply unit 32 that supplies air thereinto, the decontamination chamber R2 includes an air supply unit 30 that supplies air thereinto, and the clean bench chamber R1 includes a decontamination chamber opening 35A that is openable and closable to carry the article into the decontamination chamber R2 therethrough, in which air flows from the decontamination chamber R2 into the clean bench chamber R1 through the decontamination chamber opening 35A during it is opened, or air flows from the isolator 3 into the decontamination chamber R2 through the isolator opening 37A during it is opened.

According to the above configuration, when the article within the clean bench chamber R1 is carried into the decontamination chamber R2 through the decontamination chamber opening 35A, air flows from the decontamination chamber R2 into the clean bench chamber R1 through the decontamination chamber opening 35A so that it is possible to carry the article into the decontamination chamber R2 while effectively suppressing contaminant from intruding from the clean bench chamber R1 into the decontamination chamber R2. Also, when the article carried from the clean bench chamber R1 and placed within the decontamination chamber R2 is carried into the isolator 3 through the isolator opening 37A, air flows from the isolator 3 into the decontamination chamber R2 through the isolator opening 37A so that it is possible to carry the article into the isolator 3, while effectively suppressing contaminant from intruding from the decontamination chamber R2 into the isolator 3.

The cell treatment apparatus according to the embodiment may be configured so that the clean bench chamber R1 includes a decontamination chamber opening 35A that is openable and closable to carry the article into the decontamination chamber R2 therethrough, the decontamination chamber R2 includes air supply and exhaust units 30 and 31 (air supply unit 30, air exhaust unit 31) that supplies air thereinto and exhausts air therefrom to the outside, and an isolator opening 37A that is openable and closable to carry the article into the isolator 3 therethrough, in which the supply of air into the decontamination chamber R2 is maintained during the isolator opening 37A is closed and the decontamination chamber opening 35A is opened, and air supplied to the decontamination chamber R2 flows toward the clean bench chamber R1 through the decontamination chamber opening 35A by stopping the exhaust of air from the decontamination chamber R2 to the outside or decreasing the amount of air exhausted from the decontamination chamber R2 to the outside.

According to the above configuration, air supplied into the decontamination chamber R2 flows toward the clean bench chamber R1 through the decontamination chamber opening 35A so that it is possible to suppress contaminant within the clean bench chamber R1 from intruding into the decontamination chamber R2. Thus, it is possible to suppress the inside of the decontamination chamber R2 from being contaminated due to contaminant coming from the clean bench chamber R1, for example, when the article is carried from the clean bench chamber R1 into the decontamination chamber R2.

The cell treatment apparatus according to the embodiment may be configured so that the clean bench chamber R1 includes a decontamination chamber opening 35A that is openable and closable to carry the article into the decontamination chamber R2 therethrough, the decontamination chamber R2 includes air supply and exhaust units 30 and 31 (air supply unit 30, air exhaust unit 31) that supplies air thereinto and exhausts air therefrom to the outside, and an isolator opening 37A that is openable and closable to carry the article into the isolator 3 therethrough, the isolator 3 includes air supply and exhaust units 32 and 33 (air supply unit 32, air exhaust unit 33) that supplies air thereinto and exhausts air therefrom to the outside, in which the exhaust of air from the isolator 3 to the outside and the supply of air into the decontamination chamber R2 are stopped or the amount of air exhausted from the isolator 3 to the outside and the amount of air supplied into the decontamination chamber R2 are decreased during the decontamination chamber opening 35A is closed and the isolator opening 37A is opened, and air supplied into the isolator 3 flows toward the decontamination chamber R2 through the isolator opening 37A by maintaining the supply of air into the isolator 3 and the exhaust of air from the decontamination chamber R2 to the outside.

According to the above configuration, air supplied into the isolator 3 flows toward the decontamination chamber R2 through the isolator opening 37A so that it is possible to suppress contaminant within the decontamination chamber R2 from intruding into the isolator 3. Thus, it is possible to suppress the inside of the isolator 3 from being contaminated due to contaminant coming from the inside of the decontamination chamber R2, for example, when the article is carried from the decontamination chamber R2 into the isolator 3.

The cell treatment apparatus according to the embodiment may be configured so that each of the isolator opening 37A and the decontamination chamber opening 35A has such a height as to allow an article having a largest dimension among the articles to pass therethrough.

The isolator opening 37A and the decontamination chamber opening 35A each have such a height as to allow the article having a largest dimension among the articles to pass therethrough in the manner mentioned above can make it possible to limit each opening to a minimum height while all the articles can be passed therethrough, which enables air flows to be desirably controlled and hence occurrence of troubles such as contamination to be more securely avoided.

### REFERENCE SIGNS LIST

1: Culture vessel
2: Incubator
2A: Casing
2B, 2C: Door
3: Isolator
4: Pass box
5: Mounting table
6, 7: First robot arm
6A: Fixed part
6B: Base part
6C: First arm
6D: Second arm
6E: Third arm
6F: Grip
8: Observation section
9: Product container
10, 11, 12: Second robot arm
13: Processing section
14: Outlet
15: Base member
16: Microscope
17: Centrifuge tube
18: Preparation tank
18A: Cap
19: Conveying apparatus
20: Auxiliary arm
20A: Fixed part
20B: Grip
21: Fixing member
22: Box
23: Large-size waste disposal part
24: Small-size waste disposal part
25: Microscope
26: Centrifuge
27: First box
28: Second box
28A, 28B, 28C: Side wall
29: First air supply unit
30: Second air supply unit
31: Second air exhaust unit
32: Third air supply unit
33: Air exhaust unit
34: Shutter
34K: Opening
35: First cover
35A: Decontamination chamber opening
36: Air cylinder
36A: Piston rod
36B: Cylinder tube
37: Second cover
37A: Isolator opening
37H: Handle
38, 39: Guide rail
38A: Top plate part
38B: Vertical plate part
38C: Horizontal plate part
40: Bracket
41: Moving body
42: Lateral shaft
43: Bearing
44: Spacing member
45: Pin
46: Upper frame body
47: Roller
48: Lower frame body
49, 50: Stopper member
51: Container
52: Packaging bag
G1, G2: Glove port
R1: Clean bench chamber
R2: Decontamination chamber

## Claims

1. A cell treatment apparatus comprising:
an isolator that has an inner space maintained in aseptic conditions and is configured to treat cells in the inner space;
a pass box that is configured to carry an article for use in treatment of cells in the inside of the isolator into the inside of the isolator;
the pass box including a clean bench chamber and a decontamination chamber;
the clean bench chamber having an opening and closing door, and being configured to decontaminate the article carried thereinto through an opening formed by opening the opening and closing door; and
the decontamination chamber having a decontamination capability and an inner space, and being configured to be able to carry the article from the clean bench chamber into the inner space of the decontamination chamber from the clean bench chamber and carry the article into the inside of the isolator.

2. The cell treatment apparatus according to claim 1,
wherein the isolator includes an air supply unit that supplies air thereinto, the decontamination chamber includes an air supply unit that supplies air thereinto,
wherein the clean bench chamber includes a decontamination chamber opening that is openable and closable to carry the article into the decontamination chamber therethrough,
wherein the decontamination chamber includes an isolator opening that is openable and closable to carry the article into the isolator therethrough, and
wherein air flows from the decontamination chamber into the clean bench chamber through the decontamination chamber opening during it is opened, or air flows from the isolator into the decontamination chamber through the isolator opening during it is opened.

3. The cell treatment apparatus according to claim 1 or 2,
wherein the clean bench chamber includes a decontamination chamber opening that is openable and closable to carry the article into the decontamination chamber therethrough,
wherein the decontamination chamber includes an air supply unit that supplies air thereinto and an air exhaust unit that exhausts air therefrom to the outside, and an isolator opening that is openable and closable to carry the article into the isolator therethrough,
wherein the supply of air into the decontamination chamber is maintained during the isolator opening is closed and the decontamination chamber opening is opened, and
wherein air supplied into the decontamination chamber flows toward the clean bench chamber through the decontamination chamber opening by stopping the exhaust of air from the decontamination chamber to the outside or decreasing the amount of air exhausted from the decontamination chamber to the outside.

4. The cell treatment apparatus according to any one of claims 1 to 3,
wherein the clean bench chamber includes a decontamination chamber opening that is openable and closable to carry the article into the decontamination chamber therethrough,
wherein the decontamination chamber includes an air supply unit that supplies air thereinto and an air exhaust unit that exhausts air therefrom to the outside, and an isolator opening that is openable and closable to carry the article into the isolator therethrough,
wherein the isolator includes an air supply unit that supplies air thereinto and an air exhaust unit that exhausts air therefrom to the outside,
wherein the exhaust of air from the isolator to the outside and the supply of air into the decontamination chamber are stopped or the amount of air exhausted from the isolator to the outside and the amount of air supplied into the decontamination chamber are decreased during the decontamination chamber opening is closed and the isolator opening is opened, and
wherein air supplied into the isolator flows toward the decontamination chamber through the isolator opening by maintaining the supply of air into the isolator and the exhaust of air from the decontamination chamber to the outside.

5. The cell treatment apparatus according to any one of claims 2 to 4,
wherein each of the isolator opening and the decontamination chamber opening has such a height as to allow an article having a largest dimension among the articles to pass therethrough.
